# EUROPEAN PATENT SPECIFICATION

(11) **EP 3 573 778 B1**
(45) Date of publication and mention of the grant of the patent: **10.11.2021**
(21) Application number: 18702240.5
(22) Date of filing: 26.01.2018
(51) Int. Cl.: B22F 1/00, B22F 1/02, B22F 9/24, A61K 49/18

(54) **NANOMATERIAL AND METHOD OF PRODUCTION OF A NANOMATERIAL FOR MEDICAL APPLICATIONS, SUCH AS MRI OR SERS**
NANOMATERIAL UND VERFAHREN ZUR HERSTELLUNG EINES NANOMATERIALS FÜR MEDIZINISCHE ANWENDUNGEN WIE ETWA MRT ODER SERS
NANOMATÉRIAU ET PROCÉDÉ DE PRODUCTION D'UN NANOMATÉRIAU POUR DES APPLICATIONS MÉDICALES, TELLES QUE L'IRM OU LA SERS

(30) Priority: 27.01.2017 EP 17305087
(43) Date of publication of application: 04.12.2019
(73) Proprietor: Universite Paris Nord, 93430 Villetaneuse (FR); Centre National de la Recherche Scientifique, 75794 Paris Cedex 16 (FR)
(72) Inventor: SPADAVECCHIA, Jolanda, 75011 Paris (FR)
(74) Representative: Demulsant, Xavier
(86) International application number: PCT/EP2018/051988
(87) International publication number: WO 2018/138280

(56) References cited:
- CN-A- 102 935 519
- CN-A- 104 258 424
- US-A1- 2016 243 049
- NICHOLLS FRANCESCA J ET AL: "DNA-gadolinium-gold nanoparticles forin vivoT1 MR imaging of transplanted human neural stem cells", BIOMATERIALS, ELSEVIER SCIENCE PUBLISHERS BV., BARKING, GB, vol. 77, 14 November 2015 (2015-11-14), pages 291-306, XP029337987, ISSN: 0142-9612, DOI: 10.1016/J.BIOMATERIALS.2015.11.021 cited in the application
- CHAICHI MOHAMMAD JAVAD ET AL: "Glucose Chemiluminescence Biosensor Based on Covalent Immobilization of Enzyme in Glutaraldehyde-Functionalized Glass Cell and Direct Coupling of Chitosan-Induced Au/Ag alloy Nanoparticles", JOURNAL OF ANALYTICAL CHEMISTRY, CONSULTANTS BUREAU. NEW YORK, US, vol. 71, no. 2, 16 February 2016 (2016-02-16), pages 163-171, XP035700940, ISSN: 1061-9348, DOI: 10.1134/S106193481602012X [retrieved on 2016-02-16]
- SHI JUN ET AL: "Hollow hydroxyapatite@nano-Au@polyelectrolyte hybrid microparticles for triple-responsive drug delivery", JOURNAL OF CONTROLLED RELEASE, vol. 213, 2015, XP029259201, ISSN: 0168-3659, DOI: 10.1016/J.JCONREL.2015.05.102

## Description

### FIELD OF THE INVENTION

The invention generally relates to nanomaterials and the use of nanomaterials for analysis, diagnosis, or medical treatments.

By "medical treatment" is meant here, inter alia, targeting systems to tumors and an add-on improving efficacy or reducing adverse effect of physical treatment against cancer or vascular defects.

By "diagnosis" is meant here, inter alia, the use of medical imaging in the process of determining the nature and circumstances of a status of an organ, and more particularly Magnetic Resonance Imaging (MRI).

By "nanomaterials" is designated here nanoparticles or materials comprising nanoparticles (e.g. liquid suspension), nanoparticles being structures having a size from a few nanometers to hundreds of nanometers.

By "analysis" is designated here, inter alia, Surface Enhanced Raman Spectroscopy (SERS), the nanomaterials of the present invention being suitable for use during preparation of substrate for SERS.

By the expression "medical treatment" is also designated here the use of nanomaterials for cancer or vascular treatment, for example in the delivery of drugs for cancer treatment, by passively and actively targeting to tumor cells and also in enhancing effect of physical treatment (such as phototherapy or thermotherapy). Specific targeting of cancer cells for imaging and treatment is thought to be a key component of cancer management in the near future. Molecular imaging with targeted MRI contrast agent had emerged as one of the promising diagnostic approaches offering high resolution depiction of pathological anatomy and detection of associated disease biomarkers.

The invention relates, more particularly, on the production of nanomaterials that can be used as paramagnetic contrast agent for MRI or as add-on improving efficacy or reducing adverse effect of physical treatment, such as thermic treatment, radio treatment, photo-treatment against cancer or vascular defects.

### BACKGROUND OF THE INVENTION

Nanoparticles are used in various imaging techniques such as MRI (e.g. Padmanabhan et al, Nanoparticles in practice for molecular-imaging applications: an overview, Acta Biomaterialia, 41, 2016, pp. 1-16).

Magnetic resonance imaging (MRI) is a non-invasive imaging technique, extensively used in the clinic, which allows for high definition pictures of a whole-organism to be obtained over extended periods of time. In medical MRI, radiologists are most interested in looking at the NMR signal from water and fat, the major hydrogen containing components of the human body. MRI contrast depends on endogenous differences in water content (proton density) and relaxation time (T1, T2) in the tissue of interest.

It can be demonstrated that the dipolar interaction between free or unpaired electrons and water molecules induces a dramatic decrease in T1 and T2. Among the metals possessing unpaired electrons, gadolinium (Gd³⁺), manganese (Mn²⁺) and iron (Fe³⁺) appear the most powerful paramagnetic compounds, with 7, 5 unpaired electrons respectively.

Several materials have been developed to enhance the image contrast and diagnostic accuracy of MRI, these materials, named contrast agents, being of two different types: superparamagnetic compounds and paramagnetic compounds. Superparamagnetic contrast agents, also named T2 agents or negative contrast agents, are usually constructed with iron oxide. Paramagnetic contrast agents, also named T1 or positive contrast agents, are using paramagnetic metal ions having unpaired electrons in their outer shell (transition and lanthanide metals). The two main and widely used compounds of this class are gadolinium (Gd³⁺) or manganese (Mn²⁺).

Because of the toxicity, even at low concentrations, of free gadolinium, it is usually bound to a chelate (a low-molecular weight organic molecule e.g. DTPA5 diethylene triamine pentaacetic acid). Both gadolinium and the ligands alone cannot be used because of their toxicity. Commonly used contrast agents based on gadolinium chelates have been sold under the following trademarks:
- Magnevist (gadopentetate dimeglumine, DTPA-Gd), a linear ionic complex of the gadolinium ion (Gd³⁺) and polyaminocarboxylate ligands, more precisely N-methylglucamine salt of the gadolinium complex of diethylenetriamine pentaacetic acid;
- Omniscan (gadodiamide, DTPA-BMS-Gd) gadolinium neutral linear complex of diethylenetriamine pentaacetic acid bismethylamide;
- Dotarem (gadoterate meglumine, DOTA-Gd) is a macrocyclic ionic complex, having empirical formula of C₂₃H₄₂O₁₃N₅Gd (anhydrous basis), CAS Registry No. 92943-93-6;
- ProHance (gadoteridol, HP-DO3A-Gd) is a neutral macrocylic gadolinium complex of 10-(2-hydroxy-propyl)-1,4,7,10-tetraazacyclododecane 1,4,7-triacetic acid, empirical formula of C₁₇H₂₉N₄O₇Gd;
and also Gadovist (gadobutrol), Optimark (gadoversetamide), Multihance (gadobenate dimeglumine), Primovist, Eovist (gadoxetate), Vasovist.

The Gd (III) chelate for clinical applications, has been divided into two major groups of cyclic (the macrocyclic ligands, e.g. DOTA and DO3A) and acrylic (the acryclic ligands, e.g. DTPA and DTPA-BMA).

Other gadolinium based magnetic resonance contrast agents for molecular imaging have been proposed, e.g. dendrimer, hybrid nanoparticles, and targeted contrast agents, and also:
- macromolecular complexes, formed by attaching Gd (III) chelates to macromolecules, slowing down the rotational motion of the complexes, thus increasing relaxivities (for example Gd³⁺-hexanedione NPs);
- biodegradable macromolecular, for excreting rapidly as low molecular-weight agents (e.g. polydisulfide Gd(III));
- liposomal particles Gd(lll) complexes including Gd-DTPA, Gd(DTPA-BMA) and Gd-DOTA have been encapsulated in the core of liposomes to prepare nano-scaled MRI contrast agents.

Nanoparticles containing gadolinium used as contrast agent are proposed in prior art, the nanoparticles being obtained by encapsulating gadolinium in the core space of the nanoparticles; carrying gadolinium on the exterior of the nanoparticle via self-assembling technologies; or carrying gadolinium in the exterior of the nanoparticle via chemical conjugation.

WO 2011/113616 discloses a nanoparticle comprising a micelle formed by an amphiphilic block-copolymer and encapsulating a gadolinium complex, the nanoparticles being used as contrast agent for MRI for breast cancer early detection.

US 2016/0051707 discloses a method for producing a metal oxide nanoparticle-based T1-T2 dual-mode MRI contrast agent that has a core of T1 contrast material and a porous shell of T2 contrast material on the core, comprising the following steps: synthesizing metal oxide nanoparticles of T1 contrast material under inert gas environment; forming an epitaxial layer of metal oxide of T2 contrast material on the surface of metal oxide nanoparticles of T1 contrast material under inert gas environment; maintaining the formation of the layer of metal oxide of T2 contrast material under dry air environment to form multilayer nanoparticles having a core and porous shell structure; and coating multilayer nanoparticles with a biocompatible polymer selected from the group consisting of biopolymers such as chitosan, elastin, hyaluronic acid, alginate, gelatin, collagen, and cellulose; and synthetic polymers such as polyethylene glycol (PEG).

Chen et al (Gadolinium conjugated PLA-PEG nanoparticles as liver targeted molecular MRI contrast agent, Journal of drug targeting, 2011, pp. 657-665) discloses spherical nanoparticle MRI contrast agent targeted to liver developed by conjugation of gadolinium chelate groups onto the biocompatible poly(l-lactide)-block-poly(ethylene glycol) (PLA-PEG) nanoparticles. PLA-PEG conjugated with diethylenetriaminopentaacetic acid (DTPA) was used to formulate PLA-PEG-DTPA nanoparticles by solvent diffusion method, and then gadolinium was loaded onto the nanoparticles by chelated with the unfolding DTPA on the surface of the PLA-PEG-DTPA nanoparticles.

One drawback of the process discloses by Chen et al is that one polymer molecule could only be link to one chelate molecule, the amount of the surface Gd being thus limited. To overcome this drawback, Liu et al (Gadolinium-loaded polymeric nanoparticles modified with Anti-VEGF as multifunctional MRI contrast agents for the diagnosis of liver cancer, Biomaterials, 2011, pp. 5167-5176*),* discloses a multifunctional polymeric nanoparticle contrast agent (Anti-VEGF PLA-PEG-PLL-Gd NP) simultaneously modified with Gadolinium-diethylenetriamine pentaacetic acid (Gd-DTPA) and anti-vascular endothelial growth factor (VEGF) antibody to deliver Gd-DTPA to the tumor area and achieve the early diagnosis of hepatocellular carcinoma (HCC).

Park et al (Gold nanoparticles functionalized by gadolinium DTPA conjugate of cysteine as a multimodal bioimaging agent, Bioorganic & Medicinal Chemistry Letters 20, 2010, pp. 2287-2291), discloses the synthesis of gold nanoparticles coated with Gd-chelate (Au@GdL), where L is a conjugate of DTPA and cysteine, these particles being obtained by the replacement of citrate from the gold nanoparticle surfaces with gadolinium chelate (GdL).

Nicholls et al (DNA-gadolinium-gold nanoparticles for in vivo T1 MR imaging of transplanted human neural stem cells, Biomaterials 77, 2016, pp. 291-306*)* discloses a gold nanoparticle conjugate that is functionalized with modified deoxythymidine oligonucleotides bearing Gd (III) chelates and a red fluorescent Cy3 moiety to visualize in vivo transplanted human neural stem cells.

Nasiruzzaman et al (Gold nanoparticles coated with Gd-chelate as a potential CT/MRI bimodal contrast agent, Bull Korean Chem Soc, 2010, pp. 1177-1181) discloses the synthesis of gold nanoparticles coated by Gd-chelate (GdL@Au), where L is a conjugate of DTPA and 4-aminothiophenol. These particles are obtained by the replacement of citrate from the gold nanoparticle surfaces with gadolinium chelate (GdL).

Darras et al (Chitosan modified with gadolinium diethylenetriaminepentaacetic acid for magnetic resonance imaging of DNAlchitosane nanoparticles, Carbohydrate polymers 80, 2010 pp. 1137-1146*)* discloses production of spherical nanoparticles with diameters in the range of 30-150 nm, using low molecular weight chitosan and covalent linkage of diethylenetriaminepentaacetic acid (DTPA) to chitosan amine groups, Gd being located preferentially in a 2-5 nm wide area surrounding the nanoparticles.

Lopez-Neira et al (Surface Enhanced Raman Scattering of Amino Acids Assisted by Gold Nanoparticles and Gd3+ Ions, The journal of physical chemistry, 2015, pp. 4127-4135*)* discloses fixation of Gd³⁺ on L-tyrosine coated gold nanoparticles.

Wen et al (Multifunctional dendrimer-entrapped gold nanoparticles for dual mode CT/MR imaging applications, Biomaterials Volume: 34 Issue 5, 2013, pp. 1570-1580*)* discloses the synthesis of gadolinium-loaded dendrimer-entrapped gold nanoparticles (Gd-Au DENPs) for dual mode computed tomography/magnetic resonance imaging applications. In this prior art document, amine-terminated generation five poly(amidoamine) dendrimers modified with gadolinium chelator and polyethylene glycol (PEG) monomethyl ether were used as templates to synthesize gold nanoparticles (AuNPs). Followed by sequential chelation of Gd(lll) and acetylation of the remaining dendrimer terminal amine groups, multifunctional Gd-Au DENPs were formed.

One manganese Mn II chelate was available for MRI as an hepatobiliary compound used for the diagnosis of liver and pancreas lesions: mangafodipir trisodium (Mn-DPDP, Teslascan^{®}). This was a contrast agent that consists of manganese and an organic ligand, fodipir (dipyridoxyl diphosphate, DPDP). Thus product has been described in 1989 (Rocklage et al, Manganese(II) N,N'-dipyridoxylethylenediamine-N,N'-diacetate 5,5'-bis(phosphate). Synthesis and characterization of a paramagnetic chelate for magnetic resonance imaging enhancement, Inorganic Chemistry 1989, 28, pp. 477-485). This Mn II contrast agent was approved by the FDA in 1997.

CN 104 258 424 A1 discloses compound nanoparticles which integrate nuclear magnetic resonance imaging and a photothermal therapy function.

### PROBLEMS TO BE SOLVED

One of the problems associated with the use of Gd(lll) or Mn(ll) is toxicity. Another problem is that the production of nanoparticles is long and complicated.

The gadolinium metal ion is toxic in vivo, gadolinium having an ionic radius similar to the calcium ion and interfering with numerous metal dependent enzymatic systems, and extremely interfering with calcium channels and proteins binding sites.

Thus, gadolinium III ions cannot be administrated directly. Free gadolinium ions accumulate in the liver, spleen, kidney and bones.

To reduce the side effects of toxic ions and prevent tissue interaction, Gd (III) ions are combined with chelating ligands. But Toxic Gd(lll) ions may still be released of some chelates via transmetallation with other metal ions such as Zn²⁺, Ca²⁺ and Cu²⁺ in the body and protonation of the ligands in the pH low which may cause the separation of scheelite within the body.

Nephrogenic fibrosing dermopathy (NFD) is an idiopathic disorder in kidney patients. In most patients with NFD, dialysis for kidney failure occurs. Gd-containing contrast agents in patients with normal kidney function are rapidly excreted from the kidney with a half-life of about two hours. However, in patients with chronic renal failure Gd-containing contrast agents have a long half-life, may be greater than 120 to 30 hours. The combination of metabolic acidosis and insufficient clearance of Gd-containing agent is present in renal failure.

Toxicity is also present for manganese contrast agent. Mangafodipir trisodium Mn-DPDP readily dissociates to yield free manganese ions. This in vivo instability of the chelate rose concerns about potential toxicity. Free manganese, in chronic exposure, causes a parkinsonism-like syndrome due to accumulation in the brain. Furthermore, a significant neurological risk is associated with manganese intoxication in subjects with liver dysfunction/hepatic encephalopathy whose ability to eliminate manganese is reduced. It can also have a depressive action on heart function.

Mn-DPDP was withdrawn from the US market in October 2003 and the European market in July 2010.

To try to overcome the drawbacks of Mn-DPDP, attempts are made to provide manganese based MRI contrast agents, see e.g. US9198988 (Kent State University).

There is still a need inter alia for nanoparticles that can be used as high-performance contrast agents with reduced toxicity. There is still a need for nanoparticles that can be used as add-on improving efficacy or reducing adverse effect of physical treatment, such as thermic treatment, radio treatment, photo-treatment against cancer or vascular defects, these nanoparticles having a reduced toxicity.

### SUMMARY OF THE INVENTION

The strategy of the inventors is to implement an innovative synthesis of NPs with extremely controlled sizes and morphologies, with chemical functions to limit the aggregation and toxicity processes.

For this purpose, different ways of research have be conducted.

The first way is to set up synthesis protocols to add polymers (PEG diacid, Collagen, Chitosan, Alginate) as a surfactant in the reaction medium. Polymers with chemical functions that make it possible both to limit the non-specific adsorption of various biological components and to expose chemical functions allowing the immobilization of molecular receptors (COOH, -NH 2, -SH). This approach is innovative because the molecules chosen, integrated in the synthesis step, will have two types of chemical functions: a function acting as a surfactant, and which will therefore be involved in the formation of NP and a function that will be exposed on the surface of the NP allowing to graft biomolecules (antibody, drug, DNA). Stability studies at different pHs were considered.

By exploiting the plasmonic properties of NPs and more particularly their ability to exalt the local electromagnetic field and consequently the Raman signal of molecules located near the surface of NPs, biomecules of interest are detected in solution. The optimization of the Raman signal requires a control of the position of the plasmon resonance and thus the geometrical parameters of the NPs. In this context, a work on the control of the morphology and the functionality of the particles has been implemented. The impact of these elaborate systems is applied to the detection and contrast imaging of in vitro / vivo biomolecules involved in different cancer pathologies. A study of geometric parameters and optical properties of NPs is conducted to optimize light absorption by NPs and their efficiency in terms of local heat sources. The chemical-physical characterization tools (UV-Vls, Raman, NMR, MET, EDX) confirm the success of the synthesis.

One object of the invention is a method according to claim 1 for producing nanomaterial product which comprises at least one hybrid nanoparticle gold-metal-polymer, the polymer comprising at least one biopolymer, the metal being chosen among: Gd, Co, Eu, Tb, Ce, Mn, Fe, Zn, Cu, the method presenting a step of reducing gold ions and metal ions, in the presence of the biopolymer, the biopolymer being used as a stabilizer agent.

The list of the metals (Gd, Co, Eu, Tb, Ce, Mn, Fe, Zn, Cu) has been chosen because the similarity in Oxidation Number and chemical characteristics. For Gd, Eu, Tb there are implicated as contrast agent.

The hybrid nanoparticle of the invention is bimetallic and presents some atoms of gold and some atoms of another metal different from gold, with a biopolymer which do not include nucleic acids.

By biopolymer is meant here a polymer that can be produced by a living organism which is natural.

Advantageously, the biopolymers of the invention do not include nucleic acids.

Advantageously, the method is realized without chemical surfactant and without chemical linker. The method of the invention does not need to use chemical surfactant and synthetic chemical linker. In other terms, the method of invention does not need to use reactive or stabilizer agent used in the methods of prior art.

The method of the invention presents very fast steps in comparison with the methods of the state of the art, and is simple to realize, in order to obtain the hybrid nanoparticles.

Another object of the invention is such a method, the reduction being realized in a presence of a biopolymer mixture which comprises at least two biopolymers.

Another object of the invention is such a method comprising the successive steps: (Ai) introduction of metal ions in the aqueous solvent which comprises AuCl4⁻, for realizing complexation between metal ions and gold ions conducted to form gold-metal complex, during a first period; (Aii) stacking, by mixing said gold-metal complex with a first biopolymer introduced in the aqueous solvent, for obtaining a gold-metal-polymer complex, during a second period; (Aiii) reduction of the metal ions and reduction of the gold ion of the gold-metal-polymer complex, by introducing the reducing agent, during a third period.

Advantageously, step Ai of introduction of metal ions in the aqueous solvent which comprises AuCl₄⁻ is done under stirring.

After centrifugation, a nanomaterial product is obtained, having an inner core with gold atom and metal complex, and a polymer outer shell (or matrix).

Another object of the invention is such a method comprising the successive steps: (Bi) staking by mixing tetrachloroauric acid HAuCl₄ with a first biopolymer for obtaining a gold polymer complex, during a first period, (Bii) complexation by addition of metal ions to the gold polymer complex for obtaining a gold-metal-polymer complex, during a second period, (Biii) reduction of metal ions and reduction of gold ions of the gold-metal-polymer complex, by introducing a reducing agent, during a third period.

Advantageously, step Bii of complexation is done with stirring.

After centrifugation, a nanomaterial product is obtained, having an inner core with gold atom and metal complex, and a biopolymer outer shell (or a matrix).

Another object of the invention is such a method comprising a step of adding a second biopolymer: between the step (Aii) and the step (Aiii), or between the step (Bi) and the step (Bii).

Advantageously, the steps of the method are conducted at room temperature.

Advantageously, the invention does not use heating.

Advantageously, the first period, the second period and the third period last each less than fifteen minutes.

Advantageously, the first biopolymer and the second biopolymer are chosen among collagen, chitosan, polyethylene glycol diacid, alginate.

Advantageously, one of the biopolymer is chosen among: collagen, polyethylene glycol diacid.

Advantageously, the metal is gadolinium and the first biopolymer is collagen.

Another object of the invention is a nanomaterial product according to claim 7 which is disclosed to be synthetized as described above, with at least one hybrid bimetallic nanoparticle gold-metal-polymer, the metal being chosen among: Gd, Co, Eu, Tb, Ce, Mn, Fe, Zn, Cu, the polymer being at least one biopolymer.

Advantageously, the hybrid bimetallic nanoparticle comprises at least two biopolymers.

Another object of the invention is a nanomaterial product as defined in present claim 7 synthetized as described above, with at least one hybrid bimetallic nanoparticle gold-metal-polymer, the metal being chosen among Gd, Co, Eu, Tb, Ce, Mn, Fe, Zn, Cu, the polymer being at least one biopolymer, ionic bonds being formed between the atom of gold and the atoms of metal.

Another object of the invention is such a nanomaterial product, the hybrid bimetalic nanoparticle comprising an inner core with at least one atom of gold and atoms of metal surrounding the atom of gold, and an external shell with the biopolymer(s).

According to some embodiments, the hybrid nanoparticle comprises an inner core with one atom of gold, and an external shell (or a matrix) with atoms of metal and the biopolymer(s).

Advantageously, the external shell (or a matrix) of the nanoproduct has a diameter comprised between 50 and 200 nm.

Advantageously, the external shell (or a matrix) of the hybrid nanoparticle has a diameter comprised between 5 and 50 nm. Here, the diameter comprises the biopolymer with the complex Gd-Au of the hybrid nanoparticle.

In some embodiments, the metal is Gd, and the biopolymer or one of the biopolymers is collagen.

In some embodiments, the metal is Gd, and the biopolymers are collagen and PEG.

In some embodiments, the metal is Gd, and the biopolymers are PEG and alginate.

In some embodiments, the metal is Gd, and the biopolymers are PEG and chitosane.

Another object of the invention is the non-therapeutic use of the nanomaterial product as presented above according to claim 14, as a substrate for Enhanced Raman Spectroscopy (e.g. SERS), or as a contrast agent for Magnetic Resonance Imaging (MRI).

Another object of the invention is the nanomaterial product as presented above, for use as a therapeutic agent or a theranostic agent as defined in claim 13 such as a thermo-therapy agent, a phototherapy agent or a radio sensitive agent. Also disclosed is the nanomaterial product for use as a radioactive agent.

The present invention is directed to address one or more of the problems set forth above. The following presents a simplified summary of the invention in order to provide a basic understanding of some aspects of the invention. This summary is not an exhaustive overview of the invention. It is not intended to identify key of critical elements of the invention or to delineate the scope of the invention. Its sole purpose is to present some concepts in a simplified form as a prelude to the more detailed description that is discussed later.

While the invention is susceptible to various modification and alternative forms, specific embodiments thereof have been shown by way of example in the drawings. It should be understood, however, that the description herein of specific embodiments is not intended to limit the invention to the particular forms disclosed.

In particular, for example, the new synthesis gives some a hybrid nanoparticles Au-Gd, with a matrix based on collagen (Col), polyethylene glycol dihydrate (PEG), alginate (ALG) and/or chitosan (CHIT) which includes the atoms of gold and gadolinium. The choice of polymers and the order in which they are added to reaction medium, modifies the final reaction, since there is a different steric and chemical competition between polymers used as surfactant and gold and gadolinium salts which form the complex. The concentration of components makes it possible to obtain a selectivity of reaction. The synthesis is carried out in an aqueous solvent at temperature resulting in nanomaterials of extremely large size and morphology chemical functions on their surface, without further functionalization steps. Electron microscopy (MET) shows the homogeneity of these hybrid nanoparticles with a difference in polymer shell (or a matrix) and metal core. The collagen is an important component of the extracellular matrix. This synthesis will be extremely important in the vascular and osteo-articular fields, as well as contrast imaging.

### BRIEF DESCRIPTION OF THE DRAWINGS

The objects, advantages and other features of the present invention will become more apparent from the following disclosure and claims. The following non-restrictive description of preferred embodiments is given for the purpose of exemplification only with reference to the accompanying drawings in which:
- Fig. 1a, 1b and 1c are views of nanomaterial according to some embodiments of the present invention;

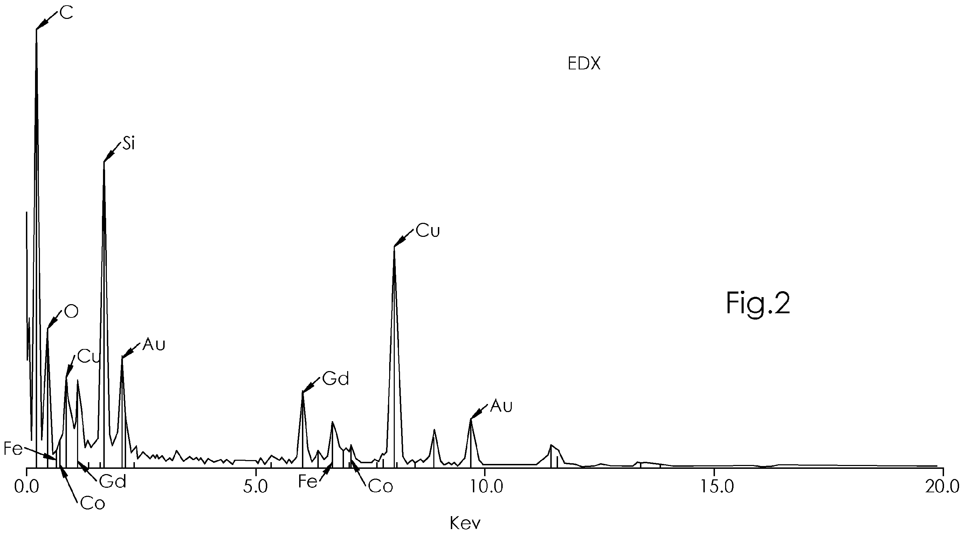

- Fig. 2 is an EDX spectrum performed on Au-Gd-polymer nanomaterial;
- Fig. 3 is a UV-VIS spectra of Au-Gd-polymer nanomaterials;
- Fig. 4 and Fig.5 are Raman spectra of Au-Gd-polymer nanomaterials;

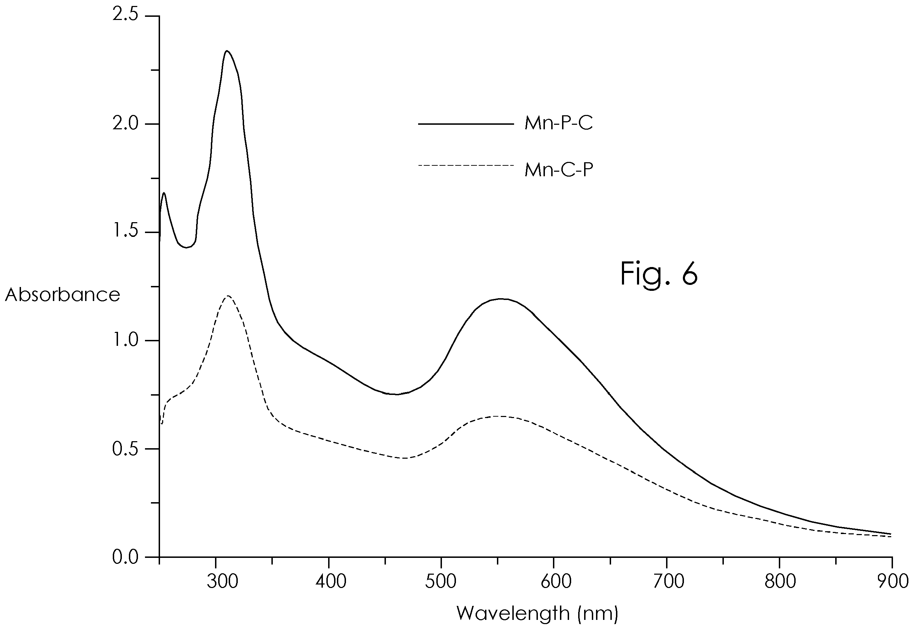

- Fig. 6 is a UV-VIS spectra of Au-Mn-polymer nanomaterials;

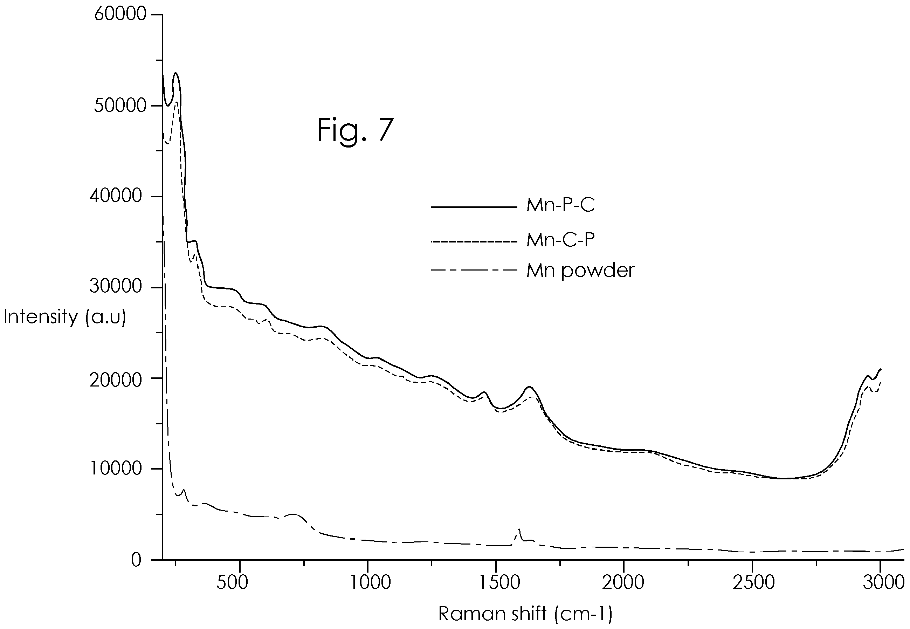

- Fig. 7 are Raman spectra of Au-Mn-polymer nanomaterials;
- Fig. 8 is a schematic of proposed mechanism of GdCl₃-AuCl₄⁻ reduction by complexation and particle formation in the presence of PEG, Col, ALG, CHIT as surfactants;
- Fig. 9 is a schematic of proposed mechanism of GdCl₃-AuCl₄⁻ reduction and particle formation in the presence of PEG, Col, ALG, CHIT;
- Fig. 10a is a Dark field and fig. 10b is a bright field schematic view (20X, 0.25 NA objective) of MIAPACA-2 cell lines treated with the GdAuNPs; the same sample region is monitored above and below;
- Fig. 11 is a schematic view of GdAuNps internalization into skin cells;
- Fig. 12 is UV-Vis Spectra of Cu AuNPs;
- Fig. 13 is a Raman Spectra of CuAuNPs; experimental conditions: λexc = 785 nm; laser power 20 mW; accumulation time 180 s;
- Fig. 14 is a UV-Vis Spectra of HAuCl₄ (full line), CuCl₂ (dashed line) and HAuCl₄-CuCl₂ (dot-dashed line) in the range 200-900nm;
- Fig. 15 a and 15 b are UV-Vis Spectras of HAuCl₄ (full line), CuCl₂ (dashed line) and HAuCl₄-CuCl₂ (dot-dashed line) in the range 200-900nm at different pH;
- Fig. 16 is a Raman Spectra of Cu-Au-Cl complex; experimental conditions: λexc = 785 nm; laser power 20 mW; accumulation time 180 s;
- Fig. 17 is a Raman Spectra of Cu-Au-Cl complex at different pH; experimental conditions: λexc = 785 nm; laser power 20 mW; accumulation time 180 s.

### DETAILED DESCRIPTION OF ILLUSTRATIVE EMBODIMENTS

### Abbreviation used

- ALG: sodium alginate;
- AuNPs: nanoparticles comprising gold;
- CHIT: chitosan (deacetylated chitin, poly(D-glucosamine));
- Col: collagen type I from calf skin;
- COSY: correlation spectroscopy;
- GdAuNPs: hybrid nanoparticles comprising gold and gadolinium;
- MnAuNPs: hybrid nanoparticles comprising gold and manganese;
- HSQC: heteronuclear single quantum coherence;
- IEFPCM polarizable continuum model using the integral equation formalism variant;
- PBS: phosphate buffer solution;
- PEG: dicarboxylic polyethylene glycol (PEG)-600;
- SEM- EDX scanning electron microscopy-energy dispersive x-ray analysis;
- TEM: transmission electron microscopy.

### Materials

Tetrachloroauric acid (HAuCl₄), gadolinium chloride hexahydrate (GdCl₃, 6H₂0), sodium borohydride (NaBH₄), PBS (pH 7,2 - 9,0), sodium chloride (NaCI), PEG, Col, CHIT, ALG were purchased from Sigma-Aldrich (Saint-Quentin Fallavier, France).

All chemicals were used as such, without further purification.

Milli Q water was used throughout the experiments.

All solvents were used without any further purification.

Experiments were carried out at room temperature, if not specified otherwise.

### Physico-chemical characterization

All the measurements were performed in triplicate, in order to validate the reproducibility of the synthetic and analytical procedures.

### UV-Vis absorption spectroscopy

Absorption spectroscopy measurements were carried out by means of a double-beam Varian Cary 500 UV-Vis spectrophotometer (Agilent, France).

Absorption spectra were recorded in the 350-900 nm spectral range in water, at AuNPs concentration equal to 10⁻⁴ M. For stability studies, Gd-AuNPs were dispersed in PBS (0, 1 M; pH 4,0 or pH 7,0), and absorption spectra collected over three months.

### TEM

TEM images were acquired with a JEOL JEM 1011 microscope (JEOL, USA) at an accelerating voltage of 100 kV.

TEM specimens were prepared after separating the PEG from the metal particles by centrifugation. Typically, 1 ml of Gd-AuNPs was centrifuged for 20 min at a speed of 14000 rpm. The upper part of the colorless solution was removed and the solid portion was re-dispersed in 1 ml of water. 2 µL of this re-dispersed particle suspension was placed on a carbon coated copper grid (manufactured by Smethurst High-Light Ltd and marketed by Agar Scientific) and dried at room temperature.

### SEM-EDX

Scanning electron microscopy (SEM) investigation was performed on an environmental SEM (ESEM, Quanta 200 FEG, FEI Company Hillsboro, OR) equipped with an (EDX) spectrometer (Genesis 2000, XMS System 60 with a Sapphire Si/Li Detector from EDAX Inc., Mahwah, NJ).

### Raman spectroscopy

The Raman experiments have been performed on an Xplora spectrometer (Horiba Scientifics-France).

The Raman spectra have been recorded using an excitation wavelength of 785 nm (laser diode) at room temperature.

For measurements in solution, a macro-objective with a focal length of 40 mm (NA = 0,18) was used in backscattering configuration. The achieved spectral resolution is close to 2 cm⁻¹.

### ¹H-NMR

Experiments were conducted on a AVANCE III spectrometer (Bruker) operating at 500 MHz with a 5 mm gradient indirect detection probe, at a probe temperature of 300 K.

64 scans were acquired to generate ID proton spectra of GdCl₃ (5 mM), PEG (1 mM) and Col, CHIT ALG products (1 mM), whereas 2048 scans were acquired for Au-Gd NP1 - Au-Gd NP5 (1 mM).

The data acquisition size was 32 K and the spectral width was 5000 Hz.

Typical 9,5 µs pulse length and relaxation delay of 2 s were used. Water signal was suppressed by applying a secondary irradiation field at the water resonance frequency (pre-saturation sequence). For each sample 500 µL aliquots were directly mixed with 100 µL Deuterium Oxide (D20) 99,96% (Eurisotop) providing an internal field-frequency lock. Samples were placed in 5 mm diameter tubes for ¹H-NMR analysis. For ¹H resonance assignment, COSY spectra were acquired for DOX with 4 K data points and 32 transients for each of the 128 increments. Moreover, 2D NMR HSQC experiments were used for ¹³C resonance assignment. Chemical shifts (δ, in ppm) were compared to the NMR solvent signal D₂0 (4,73 ppm at 300K).

### Zeta potential measurements

The zeta potential of Gd-AuNPs dispersed in water was measured using the electrophoretic mode of a Zetasizer NANOZS (Malvern Instruments Ltd, UK).

### Preparation of collagen solution (Col)

Collagen solution (Col) was diluted in PBS (pH 7,2) for 1h at room temperature.

### Preparation of Alginate solution (ALG)

10 mg of ALG was dissolved in 10 mL of PBS (pH 9,0) under ionic strength conditions (NaCl: 0.5mM) for 1h at room temperature until homogeneous solution.

### Preparation of Chitosane solution (CHIT)

10 mg of CHIT was dissolved in 10 mL of PBS (pH 9,0) under ionic strength conditions (NaCl: 0.5mM) for 1h at room temperature until homogeneous solution.

### Preparation of HAuCl₄ 6H₂O solution

16 mg of HAuCl₄ 6H₂O was dissolved in 50mL of MilliQ water at room temperature until homogenous solution.

### Preparation of GdCl₃ (Gd) solution

Solution a) 50 mg of GdCl₃*6H₂0 was dissolved in 50 mL of HAuCl₄ solution (9.4*10⁻⁴M) at room temperature until homogeneous solution. Solution b) 16 mg of GdCl_{3*}6H₂0 was dissolved in 50 mL of MilliQ water at room temperature until homogeneous solution.

### Preparation of MnCl₂ solution

16 mg of MnCl_{2*}6H₂0 was dissolved in 50 mL of MilliQ water at room temperature until homogeneous solution.

### Synthesis Procedures

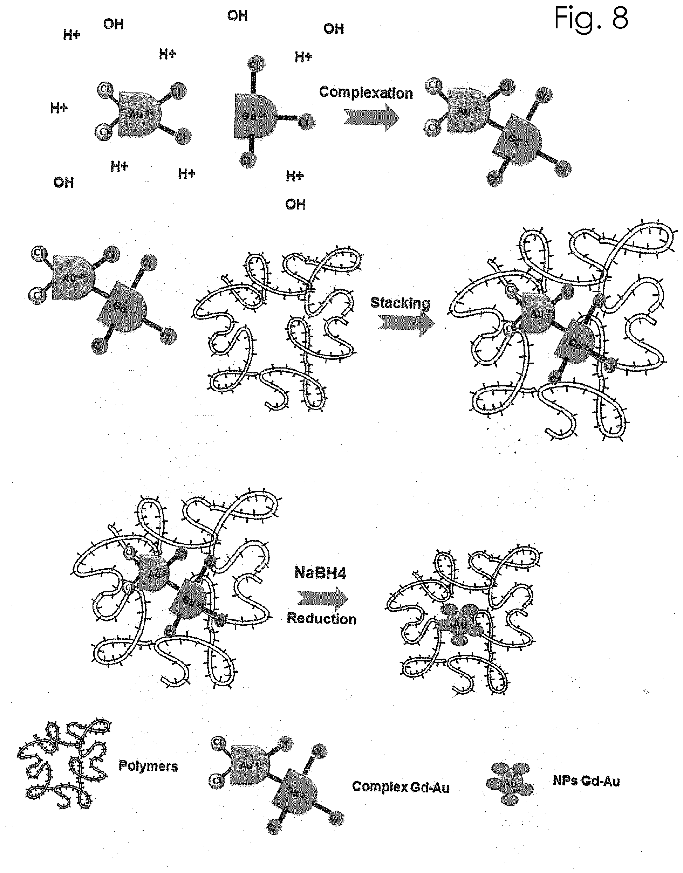

Procedures are described know with reference to the protocol represented on figure 8.

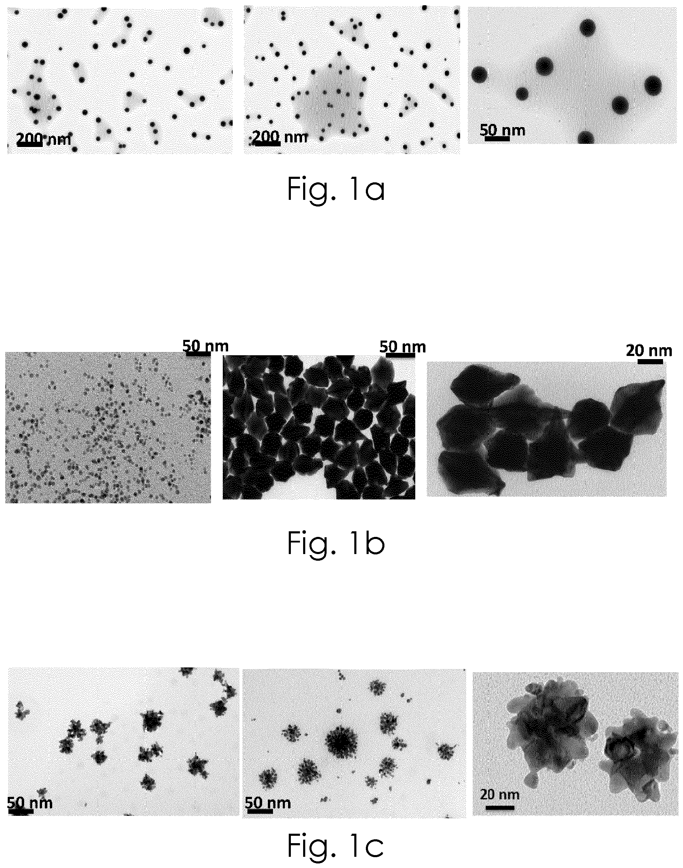

### Synthesis of Gd-AuNP1 (Gd-Au-CoINPs), see figure 1a

Gd-Au-CoINPs were prepared by a fast steps chemical process.

1ml of Gd solution b) was mixed with 20 ml of 0.0001M aqueous HAuCl₄ solution for 10 min. After this time, 1ml of Col solution was added in the resulting solution for 10 min. Then 6 ml (and/or 1.2 ml) of 0.01M NaBH₄ was added drop-wise to the resulting solution, followed by rapid stirring. After 15 min without agitation, the solution became brune-pale. The product was centrifuged at 9000 rpm for 25 min and the pellet was recovered and purified by washing with milli Q water for three times. The entire synthesis takes less than two hours.

### Synthesis of Gd-AuNP2 (Gd-Au-PEG-CoINPs), see figure 1b

2ml of Col solution was mixed with 20 ml of 0.0001M aqueous HAuCl₄ solution for 10 min. Then 250µl of PEG solution was mixed in the resulting solution under stirring at room temperature. To the resulting solution 6 ml (and/or 1.2ml) of NaBH₄ (0.01M) was added dropwise followed by rapid stirring. After 15min without agitation, the solution became red-violet. The product was centrifuged and purified at the same conditions. The entire synthesis takes less than two hours.

### Synthesis of Gd-AuNP3 (Gd-Au -PEGNPs) see figure 1c

Gd-Au-PEGNPs were prepared under two followed protocol.
1) 1ml of Gd (solution b) was mixed with 20 ml of 0.0001M aqueous HAuCl₄ solution for 10 min. After this time, 250 µl of PEG solution was added under stirring at room temperature in the resulting solution. After 10 min, 3mL (and/or 1,2 ml) of NaBH₄ (0.01 M) was added drop-wise followed by rapid stirring and kept without agitation for 2h. The resulting red-rose solution was centrifuged and purified at the same conditions.
2) Gd solution a) was dissolved in 50 mL of HAuCl₄ solution (9.4*10⁻⁴ M) at room temperature until homogeneous solution. After this time, 250 µl of PEG solution was added under stirring at room temperature in the resulting solution. After 10 min, 3mL (and/or 1,2 ml) of NaBH₄ (0.01M) was added drop-wise followed by rapid stirring and kept without agitation for 15min. The resulting red-rose solution was centrifuged and purified at the same conditions.

### Stability of Gd-AuNPs as a function of pH

The stability of hybrid nanoparticles was detected by UV VIS. All hybrid nanoparticles were dissolved in PBS solution 0.1M at different pH (pH 1, 6, 7, 8, 12) during 18h.

### Synthesis of Mn-AuNP1 (Mn-Au-PEG-CoINPs)

5 ml of Mn solution was mixed with 20 ml of 0.0001M aqueous HAuCl₄ solution for 10 min. After this time, 250 µl of Polyethylene glycol 600 Diacid (PEG) was added in the resulting solution under stirring for 5 min. Then 500µl of Collagen was added under stirring at room temperature. After 10 min, 1,2 ml of NaBH₄ (0.01M) was added drop-wise followed by rapid stirring and kept without agitation for 2h. The resulting red-rose solution was centrifuged and purified at the same conditions

### Synthesis of Mn-AuNP2 (Mn-Au-PEG-Col-ALGNPs)

5 ml of Mn solution was mixed with 20 ml of 0.0001M aqueous HAuCl₄ solution for 10 min. After this time, 0.5ml of Collagen and 1ml of ALG solutions were added in the resulting solution under stirring at room temperature. After 10 min, 1,2 ml of NaBH₄ (0.01M) was added drop-wise followed by rapid stirring and kept without agitation for 2h. The resulting red-rose solution was centrifuged and purified at the same conditions

### Synthesis of Mn-AuNP2 (Mn-Au-PEG-Col-CHITNPs)

5 ml of Mn solution was mixed with 20 ml of 0.0001M aqueous HAuCl₄ solution for 10 min. After this time, 1ml of Collagen and 1ml of CHIT solutions were added in the resulting solution under stirring at room temperature. After 10 min, 1,2 ml of NaBH₄ (0.01M) was added drop-wise followed by rapid stirring and kept without agitation for 2h. The resulting red-rose solution was centrifuged and purified at the same conditions

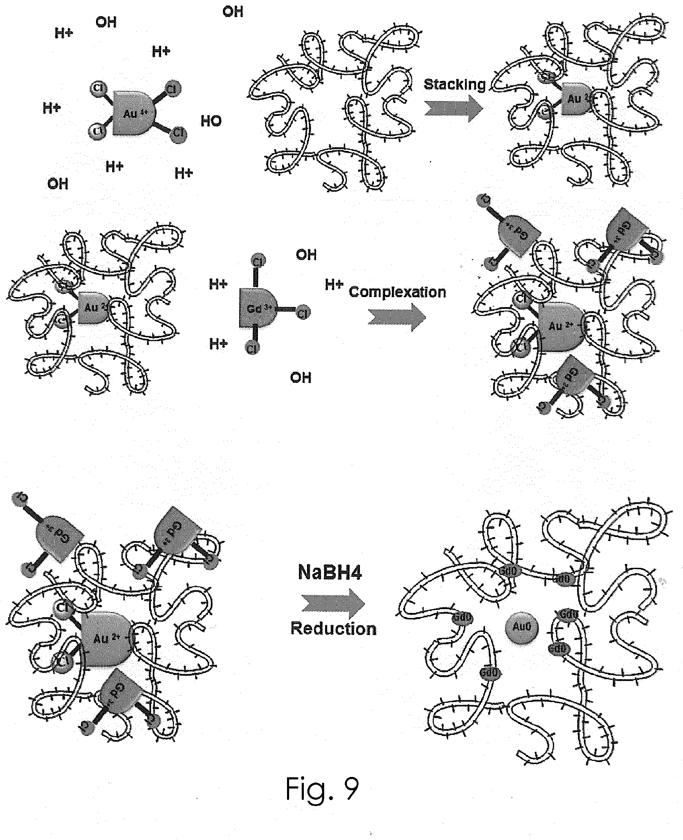

Procedures are described know with reference to the protocol represented on figure 9.

### Synthesis of Gd-AuNP1 (Gd-Au-PEG-ColNPs)

250 µl of Polyethylene glycol 600 Diacid (PEG) was mixed with 20 ml of 0.0001M aqueous HAuCl₄ solution under stirring for 10 min. After this time, 500µl of Collagen and 1 ml of Gd (protocol b) were added in the resulting solution under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Gd-AuNP2(Gd-Au-PEG-Col-ALGNPs)

250 µl of Polyethylene glycol 600 Diacid (PEG) was mixed with 20 ml of 0.0001M aqueous HAuCl₄ solution under stirring for 10 min. After this time, 50µl of Collagen, 1mL of ALG solution and 1 ml of Gd solutions were added in the resulting solution under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Gd-AuNP3(Gd-Au-PEG-Col-CHITNPs)

Gd-Au-PEG-Col-CHITNPs were prepared under same protocol of Gd-AuNP2.

250 µl of Polyethylene glycol 600 Diacid (PEG) was mixed with 20 ml of 0.0001M aqueous HAuCl₄ solution under stirring for 10 min. After this time, 500µl of Collagen, 1mL of CHIT solution and 1 ml of Gd solutions were added in the resulting solution under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Mn-AuNP1 (Mn-Au-PEG-CoINPs)

250 µl of Polyethylene glycol 600 Diacid (PEG) was mixed with 20 ml of 0.0001M aqueous HAuCl₄ solution under stirring for 10 min. After this time, 500µl of Collagen, and 2 ml of Mn solutions were added in the resulting solution under stirring at room temperature. After 10 min, 3mL of NaBH₄ (0.01M) was added drop-wise followed by rapid stirring and kept without agitation for 2h. The resulting red-rose solution was centrifuged and purified at the same conditions.

### Synthesis of Mn-AuNP2 (Mn-Au-PEG-Col-ALGNPs)

250 µl of Polyethylene glycol 600 Diacid (PEG) was mixed with 20 ml of 0.0001M aqueous HAuCl₄ solution under stirring for 10 min. After this time, 50µl of Collagen, 1mL of ALG solution and 2 ml of Mn solutions were added in the resulting solution under stirring at room temperature. After 10 min, 3mL of NaBH₄ (0.01M) was added drop-wise followed by rapid stirring and kept without agitation for 2h. The resulting red-rose solution was centrifuged and purified at the same conditions

### Synthesis of Mn-AuNP2 (Mn-Au-PEG-Col-CHITNPs)

250 µl of Polyethylene glycol 600 Diacid (PEG) was mixed with 20 ml of 0.0001M aqueous HAuCl₄ solution under stirring for 10 min. After this time, 500µl of Collagen, 1mL of CHIT solution and 2 ml of Mn solutions were added in the resulting solution under stirring at room temperature. After 10 min, 3mL of NaBH₄ (0.01M) was added drop-wise followed by rapid stirring and kept without agitation for 2h. The resulting red-rose solution was centrifuged and purified at the same conditions.

### Results and discussion

Change in the surface charge (z-potential) for each Au-Gd-NP in mv (+- standard deviation) are as follows

| | |
|---|---|
| Au-Gd-NP1 | -12.8 (+-0.2) |
| Au-Gd-NP2 | -14.8 (+-2.0) |
| Au-Gd-NP3 | -13.8 (+-1.0) |

Formation mechanism of hybrid Gd-Au NPs and role of polymers as stabilizer agent.

The synthesis of Gd-AuNPs (NP1-NP3) was carried out by reducing tetraclororoauric acid (HAuCl₄) in the presence of GdCl₃ and different biopolymers (PEG, Col, ALG, CHIT) using sodium borohydride (NaBH₄) as a reducing agent in a resulting solution by using two protocols in order to show the critical importance of order of reagents and concentrations for the shape, organization, morphology of the nanoproduct and the hybrid nanoparticles of the nanoproduct. Particles formation and growth were controlled by the amphiphilic (dual nature) character of the polymers.

In the case of the process represented on figure 8, Gd-Au-polymer modification onto the surface of AuNPs, solutions was prepared to this end. Particles formation and growth were controlled by the amphiphilic character of the polymers and includes three steps: complexation between GdCl₃ and AuCl⁴⁻ to form gold-gadolinium clusters; adsorption (stacking) of COOH-terminated polymer molecules onto Gd-Au complex; reduction of metal ions in that vicinity, growth of gadolinium-gold particles and colloidal stabilization.

In the latter case, Gd-Au complex molecules are expected to be involved in the nucleation process and may, thus, influence the final shape and size of hybrid nanoparticles.

In the case of the process represented on figure 9, the formation of golf hybrid nanoparticles from AuCl⁴⁻ can be summarized in three steps : formation of polymer mixture (i.e. PEG, Col, CHIT, ALG); initial reduction of metal ions facilitated by dicarboxylic acid-terminated PEG and/or Col to form gold clusters; adsorption (stacking) of polymers molecules on the surface of gold clusters and reduction of metals in that proximity and growth of gold particles and colloidal stabilization by molecules of polymers.

As can be seen on figures 8 and 9, depending on the process used (complexation / stacking / reduction or stacking / complexation / reduction), the metal ions (Gd, Eu, Tb, Ce, Mn, Fe, Zn, Cu) are not at the same distance to the atom of gold.

Gd (or the other metals: Co, Eu, Tb, Ce, Mn, Fe, Zn, Cu when used instead of Gd) is close to Au in the core of the hybrid nanoparticle the outer shell (or a matrix) being essentially based on polymer (figure 8). Gd being not covalent linked with the gold atom on figure 8.

On the contrary, Gd is placed at a larger distance to Au in the outer polymer shell (or a matrix) of the hybrid nanoparticle (figure 9), Gd is more far from Au in the figure 9 than in the figure 8. Gd being not covalent linked with the gold atom on figure 9.

In the present method, reduction of the salts is made in an aqueous solvent, salts of metal being chosen from a list comprising Gd, Eu, Tb, Ce, Mn, Fe, Zn, Cu, in the presence of a polymer, to obtain complexes, before obtaining hybrid nanoparticles realized after reduction. This method forms ionic bonds (i.e. electrostatic weak bonds) between the atom of gold and the atoms of metal in the created hybrid nanoparticle for the two mechanisms illustrated on figure 8 and on figure 9, whereas covalent bonds (strong bonds) are forms in prior art methods between the atom of gold and the atom of metal in view of the use of linkers or the like, for obtaining gold hybrid nanoparticles.

Interactions between AuCl₄⁻ ions and mixtures of polymers molecules (attractive ion-dipole interactions versus repulsive interactions due to hydrophobicity) are important in controlling the competition between the reactivity of AuCl₄⁻ reduction both in the bulk solution, which causes the nucleation of gold seeds.

The addiction of GdCl₃ to the gold-polymer complex induce a migration of Gd³⁺ ions inside polymer chains and consequent reduction to Gd²⁺.

Reduction with sodium borohydride NaBH₄ to obtain final hybrid nanoparticles

This result is likely due to the strong competition between PEG diacid and other polymers during reduction process of HAuCl₄

### Characterization of Gd-Au NPs

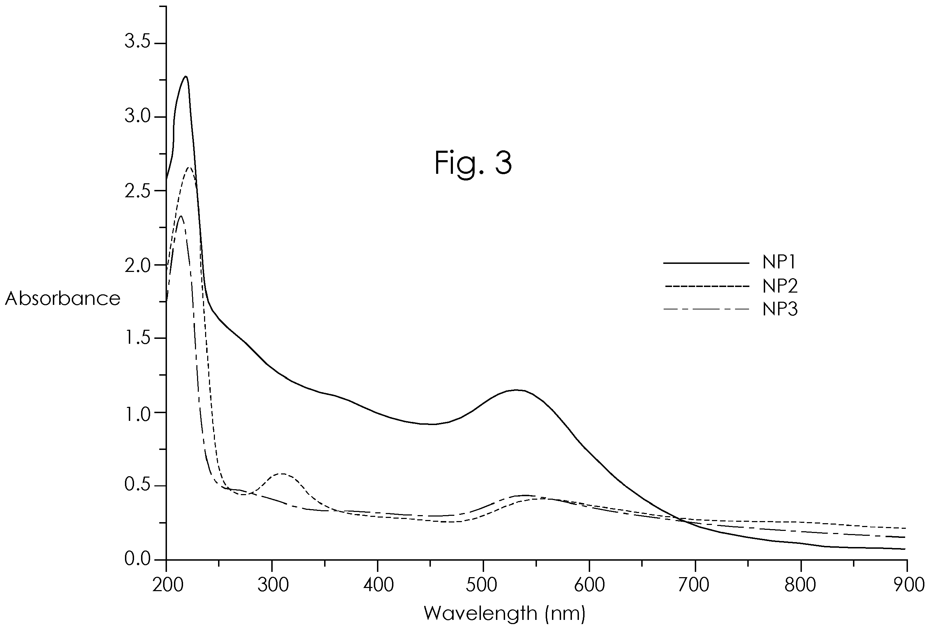

Figure 3 report absorption spectra of hybrid Gd- AuNPs, all characterized by a small peak at 300 nm and a surface plasmon band in the range 530-550 nm. The slow shift of the band position depends on the ratio of the gold salt and the capping materials during the reaction processes.

Gd-AuNP1 (Gd-Au-Col) shows a plasmon peak at 532 nm. This peak is generally ascribed to collective oscillation, known as the surface plasmon oscillation of the metal electrons in the conduction band, due to interaction of electrons with light of that wavelength. Col can be used as stabilizing polymers for AuNPs because of the dispersed solutions could be obtained due to the formation of coordination bands between Au and Gd ions with the amine or carboxylic groups respectively.

The absorption peak due at Gd-AuNP2 (Gd-Au-PEG-Col) is centered at 545 nm. This chelation evenly better dispersed Au ions and Gd which were reduced to form single AuNPs of relatively uniform size.

Gd-AuNP3 (Gd-Au-PEG) shows a strong resonance band at around 300 nm and a weaker one at 520 nm.

The shape of the nanomaterial can be modified depending on the concentrations of reagents and protocol used in the synthesis. As an example, NP1 obtained by the process of figure 8 shows hybrid gold spheres with a diameter of around 50nm (see figure 1a), whereas NP3 obtained by the process of figure 8 shows snows shape particles of about 80nm (see figure 1c), possibly due to the presence of CHIT in the growth process.

The presence of Gd ions enhance the Raman signal of biomolecules, e.g. protein or natural polymer capped onto gold hybrid nanoparticles.

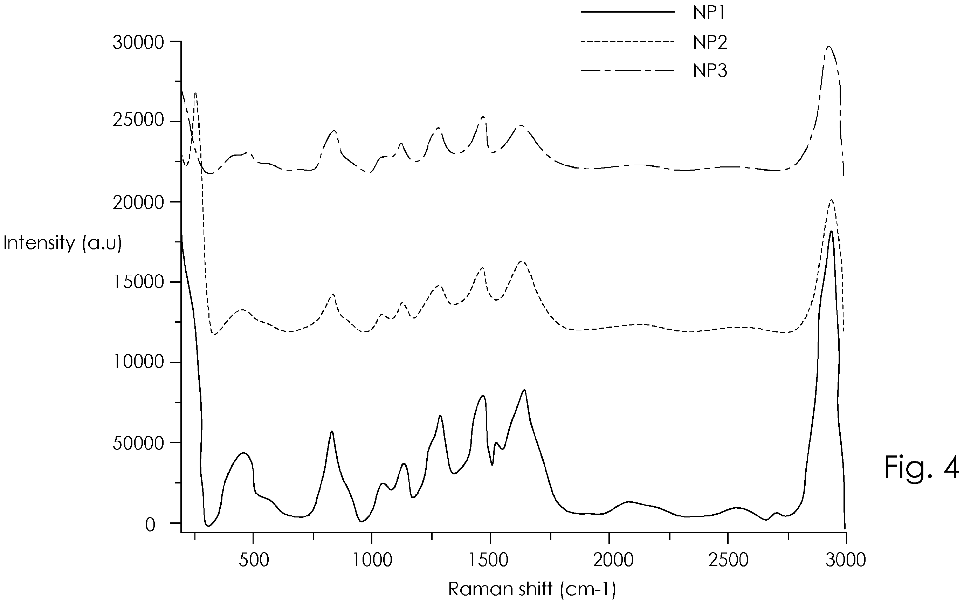

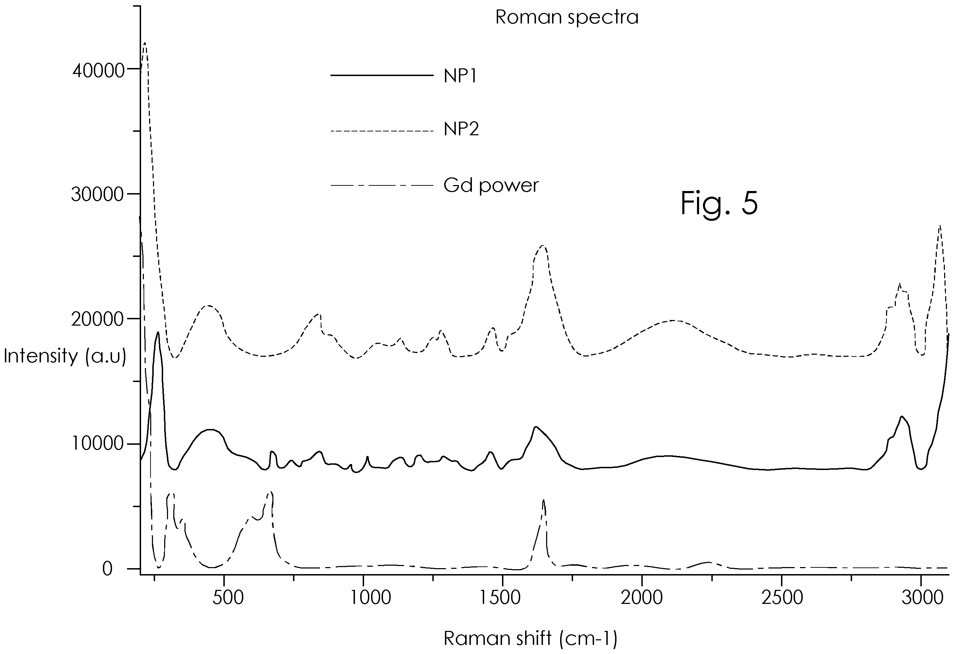

Raman spectra for powder GdCl₃ and GdAuNPs at 4.5 10⁻⁴ M are show in figures 4 and 5. The region from 1200 to 1550 cm⁻¹ corresponds to vibrations of N-H bending and C-N stretching, while the region between 1550 and 1750 cm⁻¹ corresponds to the C=O stretching mode. The band observed near 836 cm⁻¹ corresponds to vibrations of the aromatic ring. This enhancement is the result of the electric field around the gold hybrid nanoparticles interacting with collagen molecules in the presence of gadolinium. The vibration mode for the amine group is located at 1630 cm⁻¹, this band showing a signal enhancement factor of around 40 times higher than that of free collagen. This increment in the Raman signal of collagen could be due to a combination of three factors:
- formation of well-defined hot spots where Gd²⁺ ions work as spacers between gold hybrid nanoparticles;
- the refractive index contrast produced by the presence of Gd²⁺ ions causing an increment in the electric field around the hybrid nanoparticle and producing a higher enhancement factor;
- a charge transfer effect between gold hybrid nanoparticles and Gd²⁺ ions.

The invention provides inter alia synthesis of Gd-AuNPs in an aqueous solvent by reducing tetraclororoauric acid (HAuCl₄) in the presence of GdCl₃ and different polymers (PEG-diacid, Col, ALG, CHIT) using sodium borohydride (NaBH₄) as a reducing agent by using two protocols. Particles formation and growth were controlled by the amphiphilic (dual nature) character of the polymers. Synthesis are easy and brief.

The invention provides nanomaterial product that can be used as a substrate for Enhanced Raman Spectroscopy, or as a contrast agent for Magnetic Resonance Imaging, or as a therapeutic agent, a theranostic agent, a radio sensitive agent, a thermo-therapy agent or a phototherapy agent. Also disclosed is a radioactive agent.

The nanomaterial product has a low toxicity. Biopolymers such as PEG, Col, CHIT, ALG are used as stabilizer agent during the production of the nanomaterial and also as support for fixing biomolecules (e.g. DNA, medical product), enabling the use of the nanomaterial for the delivery of drugs for cancer treatment, by passively and actively targeting to tumor cells.
The method presents very fast previous (or extratemporeanous) steps (10 minutes), before the step of reduction, to obtain hybrid nanoparticles. In this sense, the method is a single step method, the single step (Aiii, Biii) being the step of reduction of the gold ions and metal ions by introducing a reducing agent, in the presence of the biopolymer, the biopolymer being used as a stabilizer agent, for obtaining a gold-metal-polymer nanoparticle.

These fast previous steps can be realized consecutively in the same receptacle.

In one first embodiment, the gold ions and the metal ions are put together (resulting solution), and ten minutes after the polymer is added in the resulting solution.

In a second embodiment, the gold ions and polymer are put together (resulting solution), and ten minutes after the metal ions are added in the resulting solution. These fast steps can be realized consecutively in the same receptacle.

### Photothermia properties

### Photothermia experiments

A 660 nm laser diode is focused on the cells and the gold nanoparticles using a 80x objective (numerical aperture = 0.75) for 10s using the optical microscope of an Xplora Raman microspectrometer (Horiba Scientifics) with a laser irradiation of 2.2 mW/µm².

### Cell treatment

Miapaca-2 cells (pancreatic cells) were exposed to a series of Gd-AuNPs dilutions in complete media (concentrations ranging from 0 to 17µM of nano) for 48h in 12 well plates. Untreated cells were also included in the experimental design. The cell were then washed, detached from the well using Trypsin-EDTA (0,25%) and resuspended in complete media. A staining with propidium iodide was added to the cell suspension at a 3µM final concentration and incubated for 15 min in the dark at room temperature. After staining with PI, cell were counted by means of BD Accuri® C6 flow cytometer, keeping constant the counting time among samples.For each sample 10 000 cells were acquired and analyzed by flow cytometry (BD Accuri™ C6 Cytometer) where FSC, SSC and FL3 data was collected.

Measurements for each sample were carried out in triplicate and are presented as median.

### Photothermal effect into PDAC cells

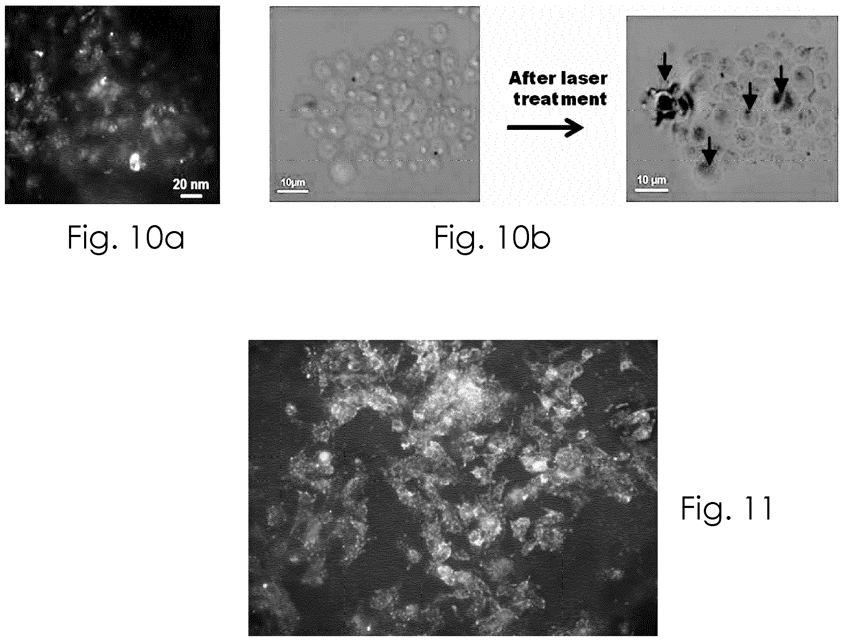

Cell internalization of the synthesized colloids (Gd-AuNPs) was checked with a confocal microscope under bright and dark field illumination. For comparison purpose, the glass slide contained regions with identically grown cells which were not exposed to the colloid solution. The images reported in Figures 10a and 10b are from the treated MIAPACA-2 cells, the same sample region is seen in dark field (fig. 10a) and in bright field conditions (fig. 10b). The dark field image shows a high density of bright, small scattering centers dispersed all over the glass slide. These dots are individual colloids or small aggregates resting on the glass slide (i.e., outside the large, microns sized cells); they are not seen under bright field due to their very small, submicrometric dimensions. Such a background of bright dots is not seen when observing the non-treated cell. Furthermore, the density of these bright spots clearly decreases if the original concentration of the incubated colloids is decreased tenfold. Within the cells, larger, bright regions are also seen (some have been marked with arrows). It appears that the colloids have a tendency to accumulate inside the cells: most of them present a central bright region. In the bright field image, dark patches, indicating lower transmission of the incident white light are seen at the corresponding positions for the larger aggregates of nanoparticles. Very similar results were obtained when treating MIAPACA-2 cells with the same batch of colloids (see Figures 10a and 10b for the relative dark field and bright field images). Individual cells were observed with a higher magnification objective (100X) to gain a better insight into the distribution of the colloidal particles.

Similar experiments were carried out after internalization of Gd AuNPs into skin cells. Figure 11 shows an example of a good internalization and homogenization in the cells. A red point confirm the presence of Gd NPs

### Synthesis Cu-Au-PEG AuNPs and description of CU-AuCL₂ complex

### Materials and Methods

### Preparation of HAuCl₄ 6H₂O (Au) solution

16 mg of HAuCl₄^{*}6H2O was dissolved in 50 mL of MilliQ water at room temperature until homogeneous solution.

### Preparation of CuCl₂ (Cu) solution

16 mg of CuCl₂ ^{*}6H2O was dissolved in 50 mL of MilliQ water at room temperature until homogeneous solution.

### Preparation of Au-CuCl₂ (Au-Cu) solution

5mL of Cu solution was added to 5 mL of Au solution and stirring during 20min

### This solution was also carried out at different pH ( 4, 7, 9)

### Syntheses of Cu-Au-NPs (Cu-Au-PEG-NPs)

250 µl of Polyethylene glycol 600 Diacid (PEG) was mixed with 20 ml of 0.0001M aqueous HAuCl₄ solution under stirring for 10 min. After this time, 5 ml of Cu solutions were added under stirring at room temperature. After 10 min, 1.2 mL of NaBH₄ (0.01M) was added drop-wise followed by rapid stirring and kept without agitation for 2h. The resulting red-rose solution was centrifuged and purified at the same conditions.

### Characterization of Cu-AuNPs

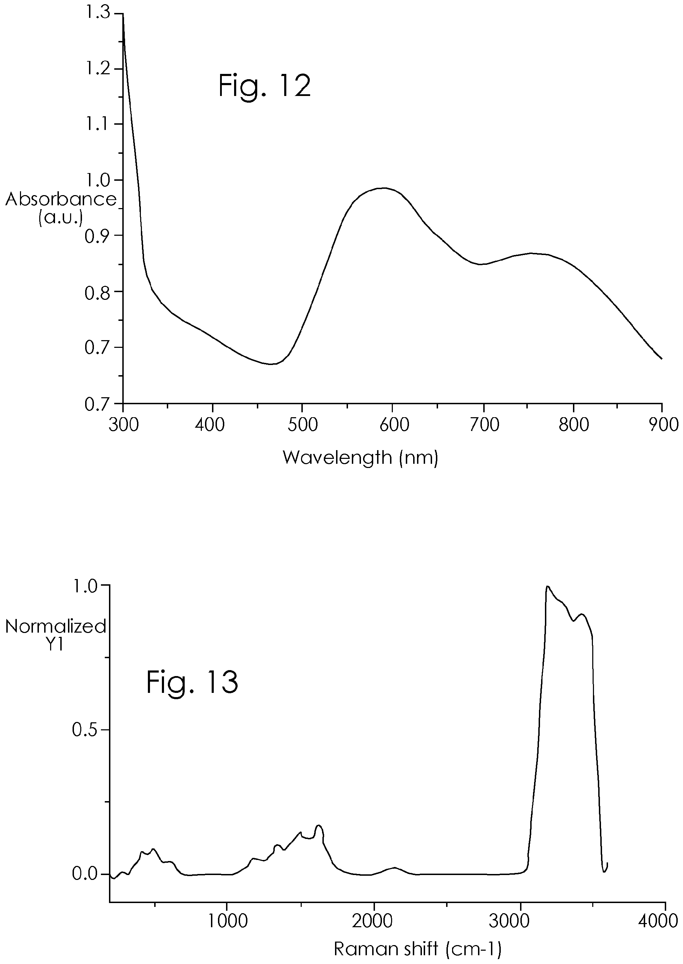

Figure 12 report absorption spectra of hybrid Cu-AuNPs, all characterized by a surface plasmon band in the range 530-560 nm and a small peak at 720 nm. The slow shift of the band position depends on the ratio of the gold salt and the capping materials during the reaction processes. Cu-AuNP1 (picture on the right) (Figure 12) shows a plasmon peak at 560 nm. This peak is generally ascribed to collective oscillation, known as the surface plasmon oscillation of the metal electrons in the conduction band, due to interaction of electrons with light of that wavelength. PEG can be used as stabilizing polymers for AuNPs because of the dispersed solutions could be obtained due to the formation of coordination bands between Au and Cu ions with the carboxylic group. This chelation evenly better dispersed Au ions and Cu which were reduced to form single AuNPs of relatively uniform size.

Figure 13 showed Raman spectra of Cu-AuNPs. In detail, Raman spectra of Cu-AuNPs exhibit many bands in the region 500-2000 cm-1 (Figure 13). The wide band observed around 1600 cm-1 on the Raman spectra is assigned to the water. Only few bands remain as the two bands around 1000 cm-1 and the one around 1620 cm-1. For instance, the strong band at 1712 cm-1 was assigned to C=O carbonyl stretching of PEG diacid. Raman spectra new bands also appear as an intense doublet at 720-760 cm-1 due to C-H plane deformation and a strong peak at 1439 cm-1 assigned to v C-C stretching. These bands are due to variation of the steric conformation of the PEG diacid, and become more prominent upon complexation with Cu-AuCl₂. As matter of fact, when C=O and hydroxyl groups of PEG diacid interacts with a metal, the sterical conformation becomes more tilted in respect to the planarone. Focusing our attention on the spectral range 200-500 cm-1(Figure 13), one can detect several spectral changes which confirms a chemical and steric modification of each drug (PTX; DTX) after complexation with gold ions and PEG-diacid molecules. One of the Raman fingerprint of the Cu-PEG-AuNPs is the presence of a band around 263 cm⁻¹ and a double peak at 235-285 cm⁻¹ was observed .These bands can be assigned to the gold chloride stretches, v (AuCI), and δ (OAuO) and δ (CuAuO) is a clear evidence of the formation of a complex between AuCl₂- and Cu and PEG diacid in solution. The common peak at 430 cm⁻¹ is due to the vibrations δ(OH...O), v(OH...O) of the PEG. The bands exhibited in the region 3000 cm⁻¹ can be assigned to aromatic C-H stretching (Figure 13). However, the vibrational frequencies exhibited at 2946-2952 cm⁻¹, are considered to be due to C-H stretching vibration of the compounds. A broad band composed of several peaks appears in the spectral range 2850-2930 cm⁻¹ is due to the symmetric CH₂-CH₃ stretch vibration of PEG-diacid molecules confirming the main role of the polymer in the synthesis of the nanoparticle.

### Mechanism of Au-Cu complex in the growth of hybrid pegylated Nanoparticles

The formation of complex Au-Cu in the growth of Nanoparticles was monitored by UV-Vis and Raman.

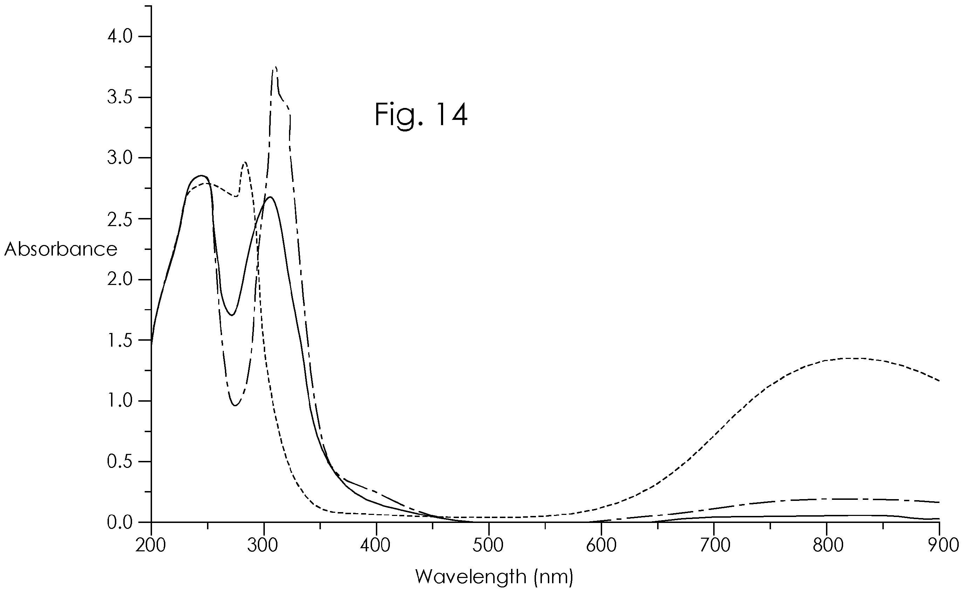

Figure 14 showed the UV-Vis fingerprint of a solution of HAuCl₄, CuCl₂ and the mixed of them (HAuCl₄+CuCl₂).

The UV-Vis Spectra showed a typical spectra of HAuCl₄ with two prominent peaks at 256 nm and 290 nm (full line in the Figure 14).

The UV-Vis spectra of CuCl₂ solution showed a peak at 256 nm, a small peak at 280 nm and a broadened peak at 800 nm (dashed line in the Figure 14).

When CuCl₂ was added to HAuCl₄ solutions, a chemical-physical modification appeared, showed in the UV Vis Spectra (dot-dashed line in Figure 14).

The principal optical modification in the spectra is due to the increase and shift of the peak from 280 nm to 320 nm due to the electronic transition. The dramatic decrease of the peat at 800 nm, confirm that the hybrid system (Au-Cu) was done.

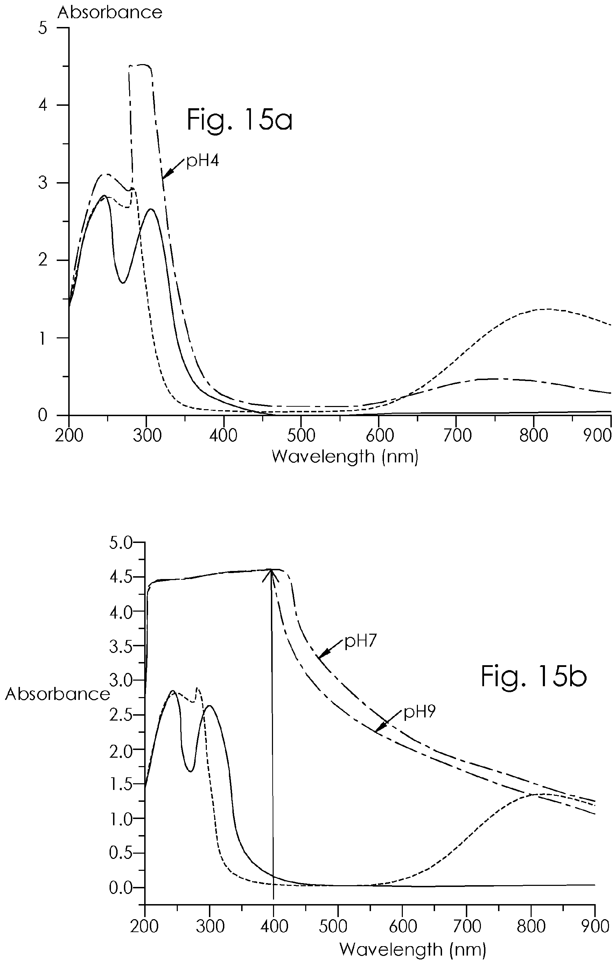

Figures 15a and 15b show LSP resonance spectra before and after incubation of HAuCl₄ mixed to CuCl₂ under specific conditions (pH: 4.0-7.0-9.0; time: 96 h).

At pH 4, we observe an increase of the peak intensity at 256 nm probably due to CuCl₂ fingerprint associated to AuCl₂ ions upon complexation was observed. The increase of the peak at 800 nm, confirming the reaction under acidic conditions.

A different behavior is observed in the case of pH 7 and pH 9 (Figure 15 b), in which after incubation at pH 7 and pH 9 for 96 h, we observe a disappearance of LSP bands. This spectroscopical behavior during pH-release gives evidence of the change of conformation of the reagents when it is encapsulated into gold nanoparticles.

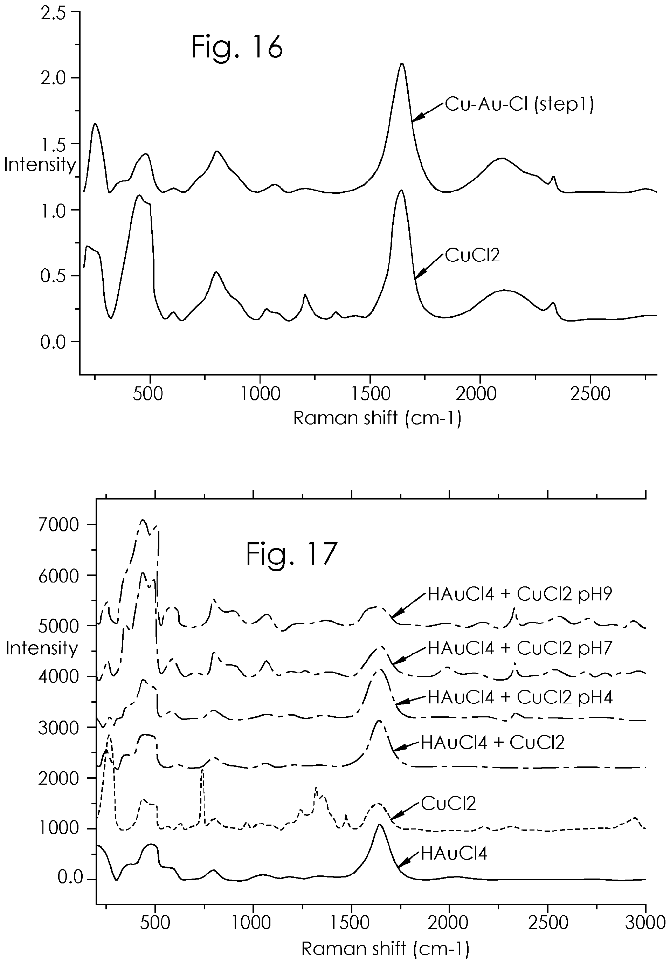

The complex Cu-Au-Cl was confirmed by Raman Spectroscopy (Figure 16), the peak at 254 cm-1 due to Cu-Au Cl and Cu-OH stretching confirm the successful of reaction

Similar experimental results were confirmed at different pH (Figure 17), in order to understand the chemical behavior of Cu and Au during complex formation in the synthesis of final nanoparticle.

## Claims

1. A method for the synthesis of the nanomaterial product of claim 7 which comprises
at least one hybrid nanoparticle of gold-metal-biopolymer,
the biopolymer comprising at least one biopolymer being chosen among: collagen, chitosan, polyethylene glycol diacid, alginate, or their derivatives,
the metal being chosen among: Gd, Co, Eu, Tb, Ce, Mn, Fe, Zn, Cu,
the method being realized in an aqueous solvent and presenting:
- Preparation steps of mixing the gold ions, the metal ions and the biopolymer, for obtaining a resulting solution with gold-metal-biopolymer complex,
- One single step of synthesizing nanomaterial product which is the reduction of gold ions and metal ions in the presence of the biopolymer, by introducing a reducing agent in the resulting solution, the biopolymer being used as a stabilizer agent,
the method being realized without chemical surfactant other than the biopolymer and without chemical linker.

2. The method according to claim 1, wherein said preparation steps, and said step of reducing the gold-metal-biopolymer complex, present each a stirring which lasts less than fifteen minutes, the method for synthesizing nanomaterial product lasts less than 2 hours.

3. A method according to claims 1 to 2, wherein the steps of the method are conducted at room temperature.

4. The method according to one of claims 1-3, comprising the following preparation steps:
- (Ai) introduction of metal ions in the aqueous solvent which comprises AuCl₄⁻, for realizing complexation between metal ions and gold ions conducted to form gold-metal complex, during a first period;
- (Aii) stacking, by mixing said gold-metal complex with a first biopolymer introduced in the aqueous solvent, for obtaining a gold-metal-biopolymer complex, during a second period.

5. The method according to claim 1-3, comprising the following preparation steps:
- (Bi) staking by mixing tetrachloroauric acid HAuCl₄ with a first biopolymer for obtaining a gold- polymer complex, during a first time period,
- (Bii) complexation by addition of metal ions to the gold- polymer complex during a second period, for obtaining a gold-metal-biopolymer complex.

6. The method according to any one of claims 4 or 5, wherein the method comprises a step of adding a second biopolymer: between the step (Aii) and the step of reduction (Aiii), or between the step (Bi) and the step (Bii).

7. A nanomaterial product, comprising at least one hybrid nanoparticle of gold-metal-biopolymer, wherein:
- The metal is selected from: Gd, Co, Eu, Tb, Ce, Mn, Fe, Zn, Cu,
- The biopolymer is selected from: collagen, chitosan, polyethylene glycol diacid, alginate, and their derivatives,
- the atom(s) of gold and the atoms of metal being linked by ionic bonds.

8. Nanomaterial product synthetized as described in claim 7, with at least one hybrid nanoparticle gold-metal-biopolymers, the metal is chosen among: Gd, Co, Eu, Tb, Ce, Mn, Fe, Zn, Cu, the hybrid nanoparticle presenting at least two different biopolymers.

9. Nanomaterial product according to one of the previous claims 7-8, wherein the hybrid nanoparticle of gold-metal-biopolymer comprises:
- an inner core with one or several atoms of gold, and atoms of metal surrounding the atom of gold; and
- an external shell with the biopolymer;

10. Nanomaterial product according to one of the previous claims 7-8, wherein the hybrid nanoparticle of gold-metal-biopolymer comprises:
- an inner core with one or several atoms of gold; and
- an external shell with atoms of metal and the biopolymer;
- the atom(s) of gold and the atoms of metal being linked by ionic bonds.

11. The nanomaterial product according to any one of claims 7 to 10, wherein the metal is Gd, and the at least one biopolymer is collagen.

12. The nanomaterial product according to claims 7 to 10, comprising two biopolymers, wherein the metal is Gd, and the biopolymers are collagen and PEG.

13. A nanomaterial product of any one of claims 7 to 12, for use as a therapeutic agent or a theranostic agent, such as:
- A thermo-therapy agent,
- A phototherapy agent, or
A radio sensitive agent.

14. Non-therapeutic use of the nanomaterial product according to anyone of claims 7 to 12, as:
- A substrate for Enhanced Raman Spectroscopy;
- A contrast agent for Magnetic Resonance Imaging;
- A radio sensitive agent.

## Patentansprüche

1. Verfahren zur Synthese des Nanomaterial-Produkts gemäß Anspruch 7, das wenigstens einen hybriden Nanopartikel aus Goldmetall-Biopolymer umfasst,
wobei das Biopolymer wenigstens ein Biopolymer umfasst, das ausgewählt ist aus: Kollagen, Chitosan, Polyethylen-Glykoldiazid, Alginat oder deren Derivaten,
wobei das Metall ausgewählt ist aus: Gd, Co, Eu, Tb, Ce, Mn, Fe, Zn, Cu,
wobei das Verfahren in einer wässerigen Lösung realisiert wird und aufweist:
Vorbereitungsschritte des Vermischens von Goldionen, Metallionen und dem Biopolymer zum Erhalten einer resultierenden Lösung mit einem Goldmetall-Biopolymerkomplex,
einen Einzelschritt des Synthetisierens eines Nanomaterial-Produkts, das die Reduktion von Goldionen und Metallionen bei Vorhandensein des Biopolymers durch Einführen eines Reduktionsmittels in die resultierende Lösung ist, wobei das Biopolymer als ein Stabilisationsmittel verwendet wird,
wobei das Verfahren ohne ein anderes chemisches Netzmittel als das Biopolymer und ohne chemischen Linker realisiert wird.

2. Verfahren gemäß Anspruch 1, wobei die genannten Vorbereitungsschritte und der genannte Reduktionsschritt des Goldmetall-Biopolymerkomplexes jeweils ein Rütteln aufweisen, das weniger als fünfzehn Minuten dauert, wobei das Verfahren zum Synthetisieren des Nanomaterialprodukts weniger als 2 Stunden dauert.

3. Verfahren gemäß Anspruch 1 bis 2, wobei die Schritte des Verfahrens bei Raumtemperatur durchgeführt werden.

4. Verfahren gemäß einem der Ansprüche 1 bis 3, umfassend die folgenden Vorbereitungsschritte:
(Ai) Einführung von Metallionen in die wässerige Lösung, die AuCl₄ umfasst, zum Realisieren einer Komplexierung zwischen Metallionen und Goldionen, die zum Bilden eines Goldmetall-Komplexes während einer ersten Periode durchgeführt wird;
(Ai) durch Mischen des genannten Goldmetall-Komplexes mit einem ersten Biopolymer, das in die wässerige Lösung eingeführt wird, Schichten zum Erhalten eines Goldmetall-Biopolymerkomplexes während einer zweiten Periode.

5. Verfahren gemäß Anspruch 1 bis 3, umfassend die folgenden Vorbereitungsschritte:
(Bi) durch Mischen von Tetrachloroaurinsure HAuCl₄ mit einem ersten Biopolymer Stützen zum Erhalten eines Goldpolymer-Komplexes während einer ersten Zeitperiode,
(Bii) Komplexierung durch Hinzufügen von Metallionen zu dem Goldpolymer-Komplex während einer zweiten Periode zum Erhalten eines Goldmetallkomplexes.

6. Verfahren gemäß irgendeinem der Ansprüche 4 oder 5, wobei das Verfahren einen Schritt des Hinzufügens eines zweiten Biopolymers umfasst: zwischen dem Schritt (Aii) und dem Reduktionsschritt (Aiii) oder zwischen dem Schritt (Bi) und dem Schritt (Bii).

7. Nanomaterial-Produkt, umfassend wenigstens einen hybriden Nanopartikel aus Goldmetall-Biopolymer, wobei:
das Metall ausgewählt ist aus: Gd, Co, Eu, Tb, Ce, Mn, Fe, Zn, Cu,
das Biopolymer ausgewählt ist aus: Kollagen, Chitosan, Polyethylen-Glykoldiacid, Alginat und deren Derivaten,
wobei das/die Atom(e) aus Gold und die Atome aus Metall durch ionische Verbindungen verbunden sind.

8. Gemäß Beschreibung in Anspruch 7 synthetisiertes Nanometall-Produkt mit wenigstens einem hybriden Nanopartikel, Goldmetall-Biopolymeren, wobei das Metall ausgewählt ist aus: Gd, Co, Eu, Tb, Ce, Mn, Fe, Zn, Cu, wobei der hybride Nanopartikel wenigstens zwei unterschiedliche Biopolymere aufweist.

9. Nanomaterial-Produkt gemäß einem der voranstehenden Ansprüche 7 bis 8, wobei der hybride Nanopartikel aus Goldmetall-Biopolymer umfasst:
einen inneren Kern mit einem oder mehreren Atom(en) aus Gold und Atome aus Metall, die das Atom aus Gold umgeben; und
eine äußere Schale mit dem Biopolymer.

10. Nanomaterial-Produkt gemäß irgendeinem der voranstehenden Ansprüche 7 bis 8, wobei der hybride Nanopartikel aus Goldmetall-Biopolymer umfasst:
einen inneren Kern mit einem oder mehreren Atom(en) aus Gold; und
eine äußere Schale mit Atomen aus Metall und dem Biopolymer;
wobei das/die Atom(e) aus Gold und die Atome aus Metall durch ionische Verbindungen verbunden sind.

11. Nanomaterial-Produkt gemäß irgendeinem der Ansprüche 7 bis 10, wobei das Metall Gd ist und das wenigstens eine Biopolymer Kollagen ist.

12. Nanomaterial-Produkt gemäß Anspruch 7 bis 10, umfassend zwei Biopolymere, wobei das Metall Gd ist und die Biopolymere Kollagen und PEG sind.

13. Nanomaterial-Produkt gemäß irgendeinem der Ansprüche 7 bis 12 zur Verwendung als ein therapeutisches Mittel oder als ein theranostisches Mittel, wie z. B.:
einen Thermotherapie-Wirkstoff,
einen Lichttherapie-Wirkstoff oder
einen funksensitiven Wirkstoff.

14. Nicht-therapeutische Verwendung des Nanomaterial-Produkts gemäß irgendeinem der Ansprüche 7 bis 12 als:
ein Substrat für eine spitzenverstärkte Raman-Spektroskopie;
ein Kontrastmittel für eine Magnetresonanz-Bildgebung;
einen funksensitiven Wirkstoff.

## Revendications

1. Procédé pour la synthèse du produit à nanomatériau de la revendication 7 qui comprend au moins une nanoparticule hybride d'or-métal-biopolymère,
le biopolymère comprenant au moins un biopolymère choisi parmi : le collagène, le chitosan, le diacide polyéthylène glycol, l'alginate, ou leurs dérivés,
le métal étant choisi parmi : Gd, Co, Eu, Tb, Ce, Mn, Fe, Zn, Cu,
le procédé étant réalisé dans un solvant aqueux et présentant :
- des étapes de préparation consistant à mélanger les ions or, les ions métal et le biopolymère, pour obtenir une solution résultante avec un complexe or-métal-biopolymère,
- une étape unique consistant à synthétiser le produit à nanomatériau qui est la réduction d'ions or et d'ions métal en présence du biopolymère, par introduction d'un agent réducteur dans la solution résultante, le biopolymère étant utilisé en tant qu'agent stabilisant,
le procédé étant réalisé sans tensioactif chimique autre que le biopolymère et sans liant chimique.

2. Procédé selon la revendication 1, dans lequel lesdites étapes de préparation, et ladite étape de réduction du complexe or-métal-biopolymère, présentent chacune une agitation qui dure moins de quinze minutes, le procédé de synthèse de produit à nanomatériau dure moins de 2 heures.

3. Procédé selon les revendications 1 et 2, dans lequel les étapes du procédé sont effectuées à température ambiante.

4. Procédé selon l'une des revendications 1 à 3, comprenant les étapes de préparation suivantes :
- (Ai) introduction d'ions métal dans le solvant aqueux qui comprend AuCl₄⁻, pour réaliser une complexation entre des ions métal et des ions or effectuée pour former un complexe or-métal, pendant une première période ;
- (Aii) empilement, par mélange dudit complexe or-métal avec un premier biopolymère introduit dans le solvant aqueux, pour obtenir un complexe or-métal-biopolymère, pendant une seconde période.

5. Procédé selon les revendications 1 à 3, comprenant les étapes de préparation suivantes :
- (Bi) empilement par mélange d'acide tétrachloroaurique HAuCl₄ avec un premier biopolymère pour obtenir un complexe or-polymère, pendant une première période de temps,
- (Bii) complexation par addition d'ions métal au complexe or-polymère pendant une seconde période, pour obtenir un complexe or-métal-biopolymère.

6. Procédé selon l'une quelconque des revendications 4 ou 5, dans lequel le procédé comprend une étape d'addition d'un second biopolymère : entre l'étape (Aii) et l'étape de réduction (Aiii), ou entre l'étape (Bi) et l'étape (Bii).

7. Produit à nanomatériau, comprenant au moins une nanoparticule hybride d'or-métal-biopolymère, dans lequel :
- le métal est choisi parmi : Gd, Co, Eu, Tb, Ce, Mn, Fe, Zn, Cu,
- le biopolymère est choisi parmi : le collagène, le chitosan, le diacide de polyéthylène glycol, l'alginate, et leurs dérivés,
- le ou les atomes d'or et les atomes de métal étant liés par des liaisons ioniques.

8. Produit à nanomatériau synthétisé comme décrit à la revendication 7, avec au moins une nanoparticule hybride d'or-métal-biopolymères, le métal est choisi parmi : Gd, Co, Eu, Tb, Ce, Mn, Fe, Zn, Cu, la nanoparticule hybride présentant au moins deux biopolymères différents.

9. Produit à nanomatériau selon l'une des revendications 7 et 8 précédentes, dans lequel la nanoparticule hybride d'or-métal-biopolymère comprend :
- un noyau intérieur avec un ou plusieurs atomes d'or, et des atomes de métal entourant l'atome d'or ; et
- une enveloppe externe avec le biopolymère.

10. Produit à nanomatériau selon l'une des revendications 7 et 8 précédentes, dans lequel la nanoparticule hybride d'or-métal-biopolymère comprend :
- un noyau intérieur avec un ou plusieurs atomes d'or ; et
- une enveloppe externe avec des atomes de métal et le biopolymère ;
- le ou les atomes d'or et les atomes de métal étant liés par des liaisons ioniques.

11. Produit à nanomatériau selon l'une quelconque des revendications 7 à 10, dans lequel le métal est Gd, et l'au moins un biopolymère est le collagène.

12. Produit à nanomatériau selon les revendications 7 à 10, comprenant deux biopolymères, dans lequel le métal est Gd, et les biopolymères sont le collagène et le PEG.

13. Produit à nanomatériau selon l'une quelconque des revendications 7 à 12, pour une utilisation en tant qu'agent thérapeutique ou agent théragnostique, tel que :
- un agent de thermo-thérapie,
- un agent de photothérapie, ou
- un agent radiosensible.

14. Utilisation non thérapeutique du produit à nanomatériau selon l'une quelconque des revendications 7 à 12, en tant que :
- substrat de spectroscopie de Raman renforcée ;
- agent de contraste d'imagerie par résonance magnétique ;
- agent radiosensible.
